(19) Europäisches Patentamt
European Patent Office
Office européen des brevets

(11) Veröffentlichungsnummer: 0 001 737
B1

(12) **EUROPÄISCHE PATENTSCHRIFT**

(45) Veröffentlichungstag der Patentschrift: 07.01.81

(51) Int. Cl.³: C 07 J 5/00, C 07 J 7/00, C 07 J 63/00

(21) Anmeldenummer: 78100483.3

(22) Anmeldetag: 24.07.78

(54) **17-Alpha-(3-Jodbenzoyloxy)-9-Alpha-chlor-4-pregnen-3.20-dione, deren D-Homo-analoga und Verfahren zu ihrer Herstellung.**

(30) Priorität: 26.10.77 DE 2748442
26.10.77 DE 2748443

(43) Veröffentlichungstag der Anmeldung: 16.05.79 Patentblatt 79/10

(45) Bekanntmachung des Hinweises auf die Patenterteilung: 07.01.81 Patentblatt 81/1

(84) Benannte Vertragsstaaten: CH FR GB NL

(56) Entgegenhaltungen:
DE-B-1 081 888
JOURNAL OF THE AMERICAN CHEMICAL SOCIETY, 99(3), 1977, Seiten 905-15

(73) Patentinhaber: SCHERING AKTIENGESELLSCHAFT Berlin und Bergkamen, Müllerstrasse 170/178 Postfach 65 0311, D-1000 Berlin 65 (DE)

(72) Erfinder: Kerb, Ulrich, Dr., Prinzregentenstrasse 7, D-1000 Berlin 31 (DE)
Erfinder: Stahnke, Manfred, Melanchthonstrasse 29, D-1000 Berlin 28 (DE)
Erfinder: Wiechert, Rudolf, Prof. Dr., Petzowerstrasse 8A, D-1000 Berlin 39 (DE)

EP 0 001 737 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).

ACTORUM AG.

# 17-Alpha-(3-Jodbenzoyloxy)-9-Alpha-chlor-4 pregnen-3.20-dione, deren D-Homo-analoga und Verfahren zu ihrer Herstellung

Die Erfindung betrifft Verbindungen gemäss Anspruch 2. Unter niederem Acyloxy sind insbesondere solche organischen Säurereste zu verstehen, die sich von niederen Alkansäuren mit bis zu 4 Kohlenstoffatomen ableiten. Genannt sei beispielsweise der Acetyl-, Propionyl-, Butyryl- und Isobutyrylrest.

Die erfindungsgemässen Verbindungen sind in erster Linie Zwischenprodukte zur Herstellung von pharmakologisch wertvollen Substanzen. Sie sind aber auch selbst schon pharmakologisch, z.B. als Antiphlogistikum, wirksam.

So erhält man aus den erfindungsgemässen Verbindungen durch Abseifen der 17α-(3-Jodbenzoyl)-gruppe in einfacher Weise, z.B. $\Delta^4$-3-Keto-9α-halogensteroide, die in der Cyclopentanophenanthren-Reihe als Verbindungen mit stark entzündungswidriger Wirkung und minimalen unerwünschten Nebenwirkungen bekannt sind (DT AS 1 081 888).

Nach Abspaltung des 9α-Chlors erhält man nach an sich bekannten Methoden, wie z.B. mit Silberperchlorat in der Wärme, mit Alkali- oder Erdalkalimetallcarbonaten in Dimethylformamid, organischen Basen wie Collidin, Lutidin und Pyridin oder mit alkoholischer Kalilauge die entsprechenden $\Delta^{9(11)}$-ungesättigten Verbindungen. Bei der letzteren Methode werden auch gleichzeitig gegebenenfalls vorhandene Estergruppen gespalten. Die so erhaltenen $\Delta^{9(11)}$-ungesättigten Pregnene sind wertvolles Ausgangsmaterial für die Herstellung von bekannten 11β-Fluorsteroiden, 11β-Hydroxy-steroiden und 9α.11β-Dihalo-D-homo-steroiden. Die 11β-Fluorsteroide werden bekanntlich so erhalten, dass man an die $\Delta^{9(11)}$-Verbindung Bromfluor addiert und aus der erhaltenen 11β-Fluor-9α-brom-Verbindung das Brom mit Tributylzinnhydrid reduziert (DT OS 2410443). Di-halo-steroide werden bekanntlich so erhalten, dass man an die $\Delta^{9(11)}$-Verbindung Bromfluor/Chlorfluor addiert (DT OS 2445817). Die 11β-Hydroxy-9α-fluor-steroide werden bekanntlich so erhalten, dass man an die entsprechende $\Delta^{9(11)}$-Verbindung unterbromige Säure zum 11β-Hydroxy-9α-bromsteroid addiert, durch Bromwasserstoffabspaltung in das 9β.11β-Oxidosteroid überführt und den Epoxidring mit Fluorwasserstoff wieder öffnet (DT AS 1026313).

Um zu den 11β-Hydroxysteroiden zu gelangen, kann man auch das 11β-Hydroxy-9α-brom-steroid mit Tributylzinnhydrid oder mit Raney-Nickel (DT AS 1179549) oder mit Chrom II-chlorid (US PS 3026337) debromieren.

Die erfindungsgemässen Zwischenprodukte haben insbesondere den Vorteil, dass sie einen leichten Zugang zu den 11β-hydroxylierten Steroiden bieten, die bekanntlich durch mikrobiologische Hydroxylierung hergestellt werden. Während bei mikrobiologischen Verfahrensschritten aufwendige Vorkehrungen getroffen werden müssen (Anzüchten der Mikroorganismen, Sterilität aller Arbeitsmittel, grosse Volumina usw.), werden die erfindungsgemässen 11β-Hydroxysteroide durch technisch einfach zu realisierende Verfahrensschritte hergestellt.

Die Erfindung betrifft des weiteren ein Verfahren zur Herstellung gemäss Anspruch 1.

Aus den Arbeiten von Breslow et al., (z.B. J. Amer Chem Soc. 96 [1974] 1973, ibid. 96 [1974] 6791) ist bekannt, dass man bei in 3α-Stellung veresterten Steroiden mit Dichlorjodbenzol unter Lichteinfluss die tertiären Kohlenstoffatome $C_5$, $C_9$ und $C_{14}$ chlorieren und anschliessend Chlorwasserstoff unter Ausbildung einer Doppelbindung wieder abspalten kann.

Dieses Verfahren hat jedoch den Nachteil, dass es allein auf solche Steroide anwendbar ist, die keine ungeschützten Carbonylgruppen, wie in 3- oder 20-Stellung, aufweisen.

Nach dem erfindungsgemässen Verfahren werden jedoch selektiv 9α-Chlor-3.20-ketopregnane erhalten.

Es war durchaus überraschend, dass die Chlorierung selektiv in 9-Stellung erfolgt, da zum einen aus dem US PS 2681353 bekannt war, dass Dichlorjodbenzol mit 20-Ketopregnanen quantitativ zu 21-Chlor-20-Ketopregnanen führt.

Zum anderen war aus den Arbeiten von Halpern (Chem. & Ind., 1962, 1571) bekannt, dass Steroide mit Doppelbindungen mit Dichlorjodbenzol zu den entsprechenden α-Dichlorsteroiden reagieren.

Das erfindungsgemässe Verfahren wird zweckmässigerweise so durchgeführt, dass man das 17α-Hydroxy-$\Delta^4$-3.20-diketopregnen in einem geeigneten Lösungsmittel wie Pyridin, Lutidin, Collidin, Benzol, Methylenchlorid, Chloroform, Äthylenchlorid, Äthylenglykoldimethyläther, mit einem 4-(disubstituierten Amino)-pyridin, wie 4-Dimethylaminopyridin, 4-Pyrrolidinopyridin, 4-Piperidinopyridin und einem 3-Jodbenzoesäurehalogenid, vorzugsweise mit dem -chlorid oder 3-Jodbenzoesäureanhydrid umsetzt.

Möglich ist aber auch eine Veresterung mit dem gemischten Anhydrid der 3-Jodbenzoesäure, das in situ durch Reaktion von Trifluoressigsäureanhydrid mit 3-Jodbenzoesäure erzeugt wird. Diese Umsetzung wird vorteilhafterweise in einem organischen Lösungsmittel wie in Benzol, Anisol, Methylenchlorid bei Temperaturen von 40 bis 100° durchgeführt.

Der so erhaltene 17α-(3-Jodbenzoyl)-steroidester wird mit Dichlorjodbenzol (Phenyljoddichlorid) in einem geeigneten Lösungsmittel photochemisch halogeniert.

Geeignete Lösungsmittel sind solche, die nicht von dem verwendeten Halogenierungsmittel angegriffen werden, wie halogenierte Kohlenwasserstoffe, z.B. Methylenchlorid, Chloroform, Trichloräthylen, Dichloräthylen und aromatische Kohlenwasserstoffe wie Benzol, Chlorbenzol und Toluol, die gegebenenfalls auch als Mischungen untereinander verwendet werden können. Zweckmässig wird die Reaktion unter Sauerstoffausschluss in einer Schutzgasatmosphäre ausgeführt. Hierzu leitet man ein inertes Gas wie Stickstoff oder Argon in die Reaktionslösung.

Die photochemische Reaktion wird durch langwelliges UV-Licht induziert, wie man es z.B. durch im Handel erhältliche Ultraviolettstrahler der Ultra-Violet Products Inc., USA oder der Quarzlampen GmbH. Hanau, Bundesrepublik, erzeugt.

Es hat sich als zweckmässig erwiesen, in Gegenwart eines basischen Puffers die Photochlorierung durchzuführen. Der basische Puffer wirkt bei der Reaktion als Chlorwasserstoffänger. Geeignet sind beispielsweise Kalium- und Natriumacetat, Natriumbicarbonat, organische Basen wie Pyridin und Morpholin.

Die nachfolgenden Beispiele sollen die Erfindung und die weitere Verwendung der erhaltenen Verbindungen verdeutlichen.

Beispiel 1

a) 3,7 g 21-Acetoxy-17α-hydroxy-4-pregnen-3.20-dion werden in 100 ml Pyridin mit 19,5 g Dimethylaminopyridin und mit 11,2 ml 3-Jodbenzoesäurechlorid in einer Argon-Atmosphäre 22 Stunden bei 80°C Badtemperatur gerührt. Das Pyridin wird im Vakuum abdestilliert, der Rückstand wird mit Wasser verrührt, in Methylenchlorid aufgenommen und der Extrakt nacheinander mit 1 N Salzsäure, Natriumhydrogencarbonat-Lösung und Wasser gewaschen. Nach dem Trocknen über Natriumsulfat wird das Lösungsmittel im Vakuum entfernt und der Rückstand an Silicagel chromatographiert. Durch Eluieren mit Methylenchlorid-Essigester erhält man 4,5 g 21-Acetoxy-17α-(3-jodbenzoyloxy)-4-pregnen-3,20-dion, das nach Umkristallisieren aus Methanol-Methylenchlorid bei 224–226°C schmilzt.
UV: $\varepsilon_{222} = 34200$.

b) 2,8 g 21-Acetoxy-17α-(3-jodbenzoyloxy)-4-pregnen-3.20-dion werden in 250 ml-Methylenchlorid gelöst und in einem Messzylinder unter Durchleiten von Argon und Zugabe von 1,8 g Jodbenzoldichlorid mit einem Ultraviolettstrahler (Type XX 15C von Ultra-Violet Products Inc., USA) bei einer Wellenlänge von 320–400 nm 15 Minuten bestrahlt. Die Reaktionslöung wird anschliessend mit 2%iger Natriumhydrogensulfit-Lösung, 2%igem Natriumhydrogencarbonat und Wasser gewaschen, über Natriumsulfat getrocknet und im Vakuum eingedampft. Nach Chromatographie an Silicagel erhält man das 21-Acetoxy-9α-chlor-17α-(3-jodbenzoyloxy)-4-pregnen-3.20-dion. F. 253–257°C (Zersetzung)

c) 630 mg 21-Acetoxy-9α-chlor-17α-(3-jodbenzoyloxy)-4-pregnen-3.20-dion werden in 60 ml Aceton gelöst, mit einer Lösung von 340 mg Silberperchlorat in 20 ml Wasser versetzt und 5 Stunden unter Rückfluss erhitzt. Anschliessend wird das Lösungsmittel im Vakuum abdestilliert, der Rückstand in Methylenchlorid aufgenommen und nacheinander mit Natriumchlorid-Lösung, Natriumhydrogencarbonat-Lösung und Wasser gewaschen, getrocknet und eingeengt. Nach Schichtchromatographie im System Methylenchlorid-Essigester 95:5 und Umkristallisation aus Methylenchlorid-Essigester erhält man das 21-Acetoxy-17-α-(3-jodbenzoyloxy)-4.9(11)-pregnadien-3.20-dion vom Schmelzpunkt 251–253°C.
UV: $\varepsilon_{222} = 36400$.

Beispiel 2

a) 4 g 17α-Hydroxy-4-pregnen-3.20-dion werden in 55 ml Dimethylformamid und 7 ml Triäthylamin gelöst, 3,5 ml Jodbenzoesäurechlorid und 2,9 g Dimethylaminpyridin zugesetzt und 20 Stunden unter Argonbegasung bei 80°C Badtemperatur gerührt. Danach wird in Eiswasser eingegossen, mit 1 N Salzsäure angesäuert, das ausgefallene Produkt abgesaugt, mit Wasser gewaschen und in Methylenchlorid gelöst. Die Methylenchlorid-Lösung wird eingedampft und der Rückstand an Silicagel chromatographiert. Durch Eluieren mit Methylenchlorid-Essigester erhält man das 17α-(3-Jodbenzoyloxy-4-pregnen-3.20-dion, das nach Umkristallisation aus Aceton/Hexan bei 184–185°C schmilzt.
UV: $\varepsilon_{222} = 37100$.

b) 1 g 17α-(3-Jodbenzoyloxy)-4-pregnen-3.20-dion werden in 100 ml Methylenchlorid gelöst, mit 700 mg Phenyljoddichlorid versetzt und 10 Minuten mit langwelligem UV-Licht bestrahlt. Es wird aufgearbeitet wie im Beispiel 1b) beschrieben. Nach Chromatographie an Silicagel und Umkristallisation aus Aceton/Hexan erhält man das 9α-Chlor-17α-jodbenzoyloxy-4-pregnen-3.20-dion vom Schmelzpunkt 213–214°C (Zersetzung).

c) 320 mg 9α-Chlor-17α-m-jodbenzoyloxy-4-pregnen-3.20-dion werden in 30 ml Aceton gelöst, mit einer Lösung von 140 mg Silberperchlorat in 10 ml Wasser versetzt und 1 Stunde unter Rückfluss erhitzt. Nach Aufarbeitung und Chromatographie, wie im Beispiel 1c) beschrieben, erhält man das 17α-m-Jodbenzoyloxy-4.9(11)-pregnadien-3.20-dion, das nach Umkristallisation aus Aceton/Hexan bei 188–189°C schmilzt.

Beispiel 3

333 mg 17α-m-Jodbenzoyloxy-4.9(11)-pregnadien-3.20-dion werden in 40 ml 5%ig methanolischer Kaliumhydroxid-Lösung unter Argon-Begasung 2 Stunden bei Raumtemperatur gerührt. Nach Neutralisieren mit Essigsäure wird im Vakuum eingeengt, in Methylenchlorid aufgenommen, mit Wasser gewaschen und eingedampft. Nach Umkristallisation aus Aceton/Hexan erhält man 210 mg 17α-Hydroxy-4.9(11)-pregnadien-3.20-dion vom Schmelzpunkt 212–213°C.

Beispiel 4

a) 2 g 21-Acetoxy-17α-hydroxy-1.4-pregnadien-3.20-dion werden in 75 ml Pyridin und 9,75 g Dimethylaminopyridin und 7 ml 3-Jodbenzoesäurechlorid unter Argon-Begasung 23 Stunden bei 80°C Badtemperatur gerührt. Nach Aufarbeitung und Chromatographie, wie im Beispiel 1a)

beschrieben, erhält man 2,3 g 21-Acetoxy-17α-(3-jodbenzoyloxy)-1.4-pregnadien-3.20-dion, das nach Umkristallisation aus Aceton/Hexan bei 213,5–215°C schmilzt.
UV: $\varepsilon_{222}$ = 38000.

b) 730 mg 21-Acetoxy-17α(3-jodbenzoyloxy)-1.4-pregnadien-3.20-dion werden in 100 ml Methylenchlorid gelöst, mit 430 mg Phenyljoddichlorid versetzt und unter Argon-Begasung 7 Minuten mit langwelligem UV-Licht bestrahlt. Nach Aufarbeitung wie im Beispiel 1b) beschrieben und Umkristallisation aus Aceton/Hexan erhält man 690 mg 9α-Chlor-21-acetoxy-17α-(3-jodbenzoyloxy)-1.4-pregnadien-3.20-dion vom Schmelzpunkt 231–232°C (Zersetzung).
UV: $\varepsilon_{222}$ = 36000.

c) 200 mg 9α-Chlor-21-acetoxy-17α-(3-jodbenzoyloxy)-1.4-pregnadien-3.20-dion werden in 20 ml Aceton gelöst, 250 mg Silberperchlorat in 6 ml Wasser zugesetzt und 16 Stunden unter Rückfluss erhitzt. Nach Aufarbeitung und Umkristallisation aus Aceton/Hexan erhält man das 21-Acetoxy-17α-(3-jodbenzoyloxy)-1.4.9(11)-pregnadien-3.20-dion vom Schmelzpunkt 209–210°C.
UV: $\varepsilon_{222}$ = 40100.

Beispiel 5

a) 21-Acetoxy-17α-hydroxy-16β-methyl-1.4-pregnadien-3.20-dion wird analog Beispiel 1a) zum 21-Acetoxy-17α-(3-jodbenzoyloxy)-16β-methyl-1.4-pregnadien-3.20-dion umgesetzt (Schmelzpunkt 208–210°C).

b) 1,2 g 21-Acetoxy-17α-(3-jodbenzoyloxy)-16β-methyl-1.4-pregnadien-3.20-dion werden in 130 ml Methylenchlorid mit 800 mg Jodbenzoldichlorid versetzt und unter Argon-Begasung 10 Minuten mit langwelligem UV-Licht bestrahlt. Nach Aufarbeitung und Kristallisation erhält man 1,05 g 9α-Chlor-21-acetoxy-17α-(3-jodbenzoyloxy)-16β-methyl-1.4-pregnadien-3.20-dion vom Schmelzpunkt 238–240°C (Zersetzung).

c) 900 mg 9α-Chlor-21-acetoxy-17α-(3-jodbenzoyloxy)-16β-methyl-1.4-pregnadien-3.20-dion werden in 100 ml Aceton gelöst, mit 800 mg Silberperchlorat in 8 ml Wasser versetzt und 16 Stunden unter Rückfluss erhitzt. Nach Aufarbeitung und Kristallisation aus Methylenchlorid-Essigester erhält man 650 mg 21-Acetoxy-17α-(3-jodbenzoyloxy)-16β-methyl-1.4.9(11)-pregnatrien-3.20-dion vom Schmelzpunkt 243–245°C.

Beispiel 6

1,5 g 9α-Chlor-21-acetoxy-17α-(3-jodbenzoyloxy)-1.4-pregnadien-3.20-dion werden in 100 ml 5%iger methanolischer Kaliumhydroxid-Lösung unter Argon-Begasung 2 Stunden unter Rückfluss zum Sieden erhitzt. Anschliessend wird mit Essigsäure neutralisiert, im Vakuum eingeengt, der Rückstand in Methylenchlorid aufgenommen, mit Wasser gewaschen und eingedampft. Nach Umkristallisation aus Aceton/Hexan erhält man das 17α-21-Dihydroxy-1.4.9(11)-pregnatrien-3.20-dion vom Schmelzpunkt 224–226°C (Zersetzung).

Beispiel 7

392 mg 9α-Chlor-21-acetoxy-17α-(3-jodbenzoyloxy)-1.4-pregnadien-3.20-dion werden in 15 ml Methanol und 15 ml Methylenchlorid gelöst, auf 0°C gekühlt und mit 1,8 ml 2 N Natriumhydroxid-Lösung versetzt. Die Lösung wird unter Argon-Begasung 30 Minuten bei 0°C gerührt. Anschliessend neutralisiert man mit Essigsäure, dampft im Vakuum ein und arbeitet auf. Nach Kristallisation aus Aceton erhält man 176 mg 9α-Chlor-17α.21-dihydroxy-1.4-pregnadien-3.20-dion vom Schmelzpunkt 229°C (Zersetzung).

Beispiel 8

50 mg 9α-Chlor-17α.21-dihydroxy-1.4-pregnadien-3.20-dion werden in 10 ml Aceton, 10 ml Tetrahydrofuran und 1 ml Wasser mit 200 mg Silberperchlorat 20 Stunden unter Rückfluss erhitzt. Nach Aufarbeitung und Kristallisation aus Aceton/Hexan erhält man das 17α.21-Dihydroxy-1.4.9(11)-pregnatrien-3.20-dion vom Schmelzpunkt 225°C (Zersetzung).

Beispiel 9

376 mg 21-Acetoxy-17α-hydroxy-4-pregnen-3.20-dion werden in 10 ml Methylenchlorid mit 5 g Dimethylaminopyridin und 0,56 ml 3-Jodbenzoesäurechlorid 17 Stunden unter Rückfluss erhitzt. Anschliessend wird mit Methylenchlorid verdünnt und nacheinander mit verdünnter Salzsäure, Natriumhydrogencarbonat-Lösung und Wasser gewaschen. Nach Entfernung des Lösungsmittels und Chromatographie an Silicagel erhält man 530 mg 21-Acetoxy-17α-(3-jodbenzoyloxy)-4-pregnen-3.20-dion vom Schmelzpunkt 224,5–226°C.

Beispiel 10

Aus 21-Acetoxy-6α-fluor-17α-hydroxy-4-pregnen-3.20-dion wird analog Beispiel 1a–b das 9α-Chlor-21-acetoxy-6α-fluor-17α-(3-jodbenzoyloxy)-4-pregnen-3.20-dion erhalten, das bei 243°C unter Zersetzung schmilzt.

Beispiel 11

Aus 9α-Chlor-21-acetoxy-17α-(3-jodbenzoyloxy)-6α-methyl-4-pregnen-3.20-dion und 9α-Chlor-6α-fluor-21-acetoxy-17α-(3-jodbenzoyloxy)-4-pregnen-3.20-dion erhält man durch alkalische Hydrolyse 6α-Methyl-17α.21-dihydroxy-4.9(11)-pregnadien-3.20-dion (Schmelzpunkt 171–173°C) und 6α-Fluor-17α.21-dihydroxy-4.9(11)-pregnadien-3.20-dion (Schmelzpunkt 217–219°C, Zersetzung).

Beispiel 12

a) 1,61 g 21-Acetoxy-17α-hydroxy-6α-methyl-4-pregnen-3.20-dion werden in 10 ml Methylenchlorid mit 1,96 g Dimethylaminopyridin und 3,82 g 3-Jodbenzoesäureanhydrid werden 5 Tage bei Raumtemperatur gerührt. Nach Aufarbeitung und Chromatographie an Silicagel erhält man

2,18 g 21-Acetoxy-17α-(3-jodbenzoyloxy)-6α-methyl-4-pregnen-3.20-dion. Schmelzpunkt 217–218°C (Aceton-Hexan).

b) 632,5 mg 21-Acetoxy-17α-(3-jodbenzoyloxy)-6α-methyl-4-pregnen-3.20-dion werden in 50 ml Methylenchlorid nach Zugabe von 1 g Kaliumacetat und 330 mg Phenyljoddichlorid 5 Minuten mit UV-Licht bestrahlt. Nach der Aufarbeitung und Chromatographie erhält man 570 mg 9α-Chlor-21-acetoxy-17α-(3-jodbenzoyloxy)-6α-methyl-4-pregnen-3.20-dion. Schmelzpunkt 248–251°C Zersetzung (Aceton).

c) 333,5 mg 9α-Chlor-21-acetoxy-17α-(3-jodbenzoyloxy)-6α-methyl-4-pregnen-3.20-dion werden in 5 ml Essigsäure mit 170 mg Silbernitrat 15 Minuten auf 100°C erhitzt. Man fällt in eiskalter Natriumchloridlösung aus, saugt ab und extrahiert das Produkt mit Methylenchlorid. Nach Umkristallisation aus Aceton erhält man 230 mg 21-Acetoxy-17α-(3-jodbenzoyloxy)-6α-methyl-pregna-4.9(11)-dien-3.20-dion.

Schmelzpunkt 246–248°C.

Beispiel 13

a) 9,4 g 21-Acetoxy-17α-hydroxy-1.4-pregnadien-3.20-dion werden in 250 ml Methylenchlorid mit 24,45 g Dimethylaminopyridin und 47,8 g 3-Jodbenzoesäureanhydrid 84 Stunden bei Raumtemperatur gerührt. Es wird ohne Aufarbeitung an Silicagel chromatographiert. Mit Toluol/Äther wird das 21-Acetoxy-17α-(3-jodbenzoyloxy)-1.4-pregnadien-3.20-dion eluiert und aus Aceton/Hexan umkristallisiert.

Schmelzpunkt 213,5–215°C. Ausbeute: 72,5% d. Th.

b) 4,932 g 21-Acetoxy-17α-jodbenzoyloxy-1.4-pregnadien-3.20-dion, 500 ml frisch destilliertes Methylenchlorid, 16 g Kaliumacetat gepulvert und über Phosphorpentoxid im Vakuum getrocknet,

2,75 g Jodbenzoldichlorid (mit $CC1_4$ und Pentan gewaschen).

In einem 500 ml Messzylinder mit Magnetrührung werden Methylenchlorid und Kaliumacetat 20 Minuten unter Argon-Einleitung gerührt, Phenyljoddichlorid zugegeben, 5 Min. gerührt und anschliessend das Jodbenzoat eingetragen. Bestrahlt wird mit dem UV-Strahler Typ XX 15 C 5 Min. lang. Da nach Dünnschichtchromatographie noch eine Spur vom Ausgangsmaterial vorhanden war, werden 250 mg Phenyljoddichlorid zugefügt und nochmal 10 min bestrahlt.

Die Lösung wird mit je 250 ml 5%iger Natriumhydrogensulfit-Lösung, 250 ml 5%iger Natriumhydrogencarbonat-Lösung und mit Wasser gewaschen, getrocknet und eingedampft. Der Rückstand (5,86 g) wird mit Pentan verrieben. Das Kristallisat 5,3 g wird direkt in die Chlorwasserstoff-Abspaltung eingesetzt. Es wird 21-Acetoxy-9α-chlor-17α-(3-jodbenzoyloxy)-1.4-pregnadien-3.20-dion vom Schmelzpunkt 231–232°C (Zersetzung) erhalten.

In einem parallelen Ansatz gleicher Dimension wurde in einem 1000 ml Messzylinder mit einem Durchmesser von 85 mm mit der Tauchlampe Hanau TQ 150 der Fa. Quarzlampen GmbH., Hanau, bestrahlt.

Ausbeute und Qualität der erhaltenen Produkte waren gleich.

Beispiel 14

5,36 g 21-Acetoxy-9α-chlor-17α-(3-jodbenzoyloxy)-1.4-pregnadien-3.20-dion werden in 80 ml Eisessig bei 100°C gelöst und 2,8 g Silbernitrat zugegeben und 45 min bei 100°C Badtemperatur gerührt. Nach dem Abkühlen auf Raumtemperatur werden 1,7 g Kaliumacetat zugegeben und die Essigsäure am Rotationsverdampfer abdestilliert. Der Rückstand wird in Methylenchlorid aufgenommen, mit NaCl-Lösung gewaschen und über eine G4-Fritte das Silberchlorid abgesaugt. Das Filtrat wird mit Natriumhydrogencarbonat-Lösung und Wasser gewaschen, getrocknet und im Vakuum eingedampft. Der Rückstand 5,19 g wird an Silicagel chromatographiert. Eluiert wird mit 1,5 l Methylenchlorid/1,5 l Methylenchlorid/Essigester 1:1. Nach Kristallisation aus Essigester werden 3,76 g 21-Acetoxy-17α(3-jodbenzoyloxy)-1.4.9(11)-pregnatrien-3.20-dion erhalten.

Schmelzpunkt 209–210°C.

Beispiel 15

a) 18,82 g 21-Acetoxy-17α-hydroxy-4-pregnen-3.20-dion werden in 100 ml Methylenchlorid mit 24,45 Dimethylaminopyridin und 47,8 g 3-Jodbenzoesäureanhydrid bei Raumtemperatur gerührt. Nach 5 Stunden ist alles gelöst und nach 64 Stunden die Umsetzung beendet.

Zur Aufarbeitung wird mit 2 l Methylenchlorid verdünnt und nacheinander mit 500 ml Wasser, 500 ml normaler Salzsäure, 500 ml Wasser, zweimal mit je 500 ml 5%iger Natriumbicarbonatlösung und nochmal mit 500 ml Wasser gewaschen. Nach dem Trocknen über Natriumsulfat und Eindampfen im Vakuum erhält man 33,9 g rohes 21-Acetoxy-17α-(3-jodbenzoyloxy)-4-pregnen-3.20-dion. Durch Umkristallisation aus Aceton werden 26,90 g Jodbenzoat erhalten.

Schmelzpunkt 224–226°C.

Aus den Mutterlaugen können durch Chromatographie noch weitere Mengen des Jodbenzoats isoliert werden.

b) 4,95 g 21-Acetoxy-17α-(3-jodbenzoyloxy)-4-pregnen-3.20-dion werden in 500 ml Methylenchlorid nach Zugabe von 10 g Kaliumacetat und 3,3 g Phenyljoddichlorid mit der Tauchlampe Hanau TQ 150 5 min bestrahlt und aufgearbeitet. Man erhält 4,9 g 9α-Chlor-21-acetoxy-17α-(3-jodbenzoyloxy)-4-pregnen-3.20-dion.

Schmelzpunkt 256–259°C (Zersetzung).

Beispiel 16

a) 4,7 g 21-Acetoxy-17aα-hydroxy-D-homo-4-pregnen-3.20-dion werden in 600 ml Methylenchlorid mit 11,7 g Dimethylaminopyridin und 6,7 ml 3-Jodbenzoesäurechlorid 22 Stunden unter Rückfluss erhitzt. Zur Aufbereitung wird das Reaktionsgemisch mit Methylenchlorid verdünnt, filtriert, das Filtrat mit verdünnter Salzsäure, Natriumhydrogencarbonat-Lösung und Wasser gewaschen, über Natriumsulfat getrocknet und eingedampft. Der Rückstand wird an Silicagel chromatographiert. Mit Methylenchlorid/Essigester eluiert man das 21-Acetoxy-17aα-(3-jodbenzoyloxy)-D-homo-4-pregnen-3.20-dion.

Schmelzpunkt 187–188°C,

UV: $\varepsilon_{221} = 36000$.

b) 1,9 g 21-Acetoxy-17aα-(3-jodbenzoyloxy)-D-homo-4-pregnen-3.20-dion werden in 150 ml Methylenchlorid gelöst und in einem Messzylinder unter Durchleiten von Argon und Zugabe von 0,92 g Jodbenzoldichlorid mit einem Ultraviolettstrahler (Type XX 15C von Ultra-Violet Products Inc. USA (320–400 nm) 7 Minuten bestrahlt. Die Reaktionslösung wird anschliessend mit 2%iger Natriumsulfit-Lösung, 2%iger Natriumhydrogencarbonat-Lösung und Wasser gewaschen, über Natriumsulfat getrocknet und im Vakuum eingedampft. Nach Chromatographie und Kristallisation aus Essigester erhält man das 21-Acetoxy-9α-chlor-17aα-(3-jodbenzoyloxy)-D-homo-4-pregnen-3.20-dion.

Schmelzpunkt 204–205°C (Zersetzung).

UV: $\varepsilon_{222} = 35200$ (Methanol).

Beispiel 17

200 mg 21-Acetoxy-9α-chlor-17aα-(3-jodbenzoyloxy)-D-homo-4-pregnen-3.20-dion werden in 20 ml Aceton und 6 ml Wasser mit 500 mg Silberperchlorat 1 Stunde unter Rückfluss erhitzt. Anschliessend wird das Lösungsmittel im Vakuum entfernt, der Rückstand in Methylenchlorid aufgenommen und nacheinander mit Natriumchlorid-Lösung und Wasser gewaschen, getrocknet und eingeengt. Der Rückstand wird aus Aceton-Hexan umkristallisiert. Man erhält so 21-Acetoxy-17aα-(3-jodbenzoyloxy)-D-homo-4.9(11)-pregnadien-3.20-dion.

Schmelzpunkt 191,5–194°C.

Beispiel 18

a) 2,6 g 17aα-Hydroxy-D-homo-1.4-pregnadien-3.20-dion werden in 100 ml Pyridin mit 8,2 g Dimethylaminopyridin und 9,2 ml 3-Jodbenzoesäurechlorid 24 Stunden bei 90°C Badtemperatur unter Argon-Begasung gerührt. Anschliessend wird das Pyridin im Vakuum abdestilliert, der Rückstand mit Wasser verrührt und mit Methylenchlorid extrahiert. Der Extrakt wird mit verdünnter Salzsäure, Natriumhydrogencarbonat-Lösung und Wasser gewaschen, über Natriumsulfat getrocknet, das Lösungsmittel im Vakuum entfernt und der Rückstand an Silicagel chromatographiert. Nach Umkristallisation aus Aceton/Hexan erhält man das 17aα-(3-Jodbenzoyloxy)-D-homo-1.4-pregnadien-3.20-dion.

b) 1,4 g 17aα-(3-Jodbenzoyloxy)-D-homo-1.4-pregnadien-3.20-dion werden in 120 ml Methylenchlorid gelöst, 0,75 g Jodbenzoldichlorid zugesetzt und mit langwelligem UV-Licht bestrahlt und aufgearbeitet wie im Beispiel 16 beschrieben. Nach Chromatographie und Kristallisation aus Aceton/Hexan erhält man das 9α-Chlor-17aα-(3-jodbenzoyloxy)-D-homo-1.4-pregnadien-3.20-dion.

Schmelzpunkt 233°C (Zersetzung).

Beispiel 19

300 mg 9α-Chlor-17aα-(3-jodbenzoyloxy)-D-homo-1.4-pregnadien-3.20-dion werden in 20 ml 5%iger methanolischer Kaliumhydroxid-Lösung unter Argon 2 Stunden unter Rückfluss erhitzt. Anschliessend wird mit Essigsäure neutralisiert, im Vakuum eingeengt und der Rückstand an Silicagel chromatographiert. Nach Umkristallisation aus Aceton/Hexan erhält man das 17aα-Hydroxy-D-homo-pregna-1.4.9(11)-trien-3.20-dion vom Schmelzpunkt 190–191°C.

UV: $\varepsilon_{239} = 15600$.

Beispiel 20

a) 1,4 g 17aα-Hydroxy-D-homo-4-pregnen-3.20-dion werden in 50 ml Pyridin mit 1,4 ml 3-Jodbenzoesäurechlorid und 12,5 g Dimethylaminopyridin 21 Stunden bei 80°C unter Argonbegasung gerührt. Nach Aufarbeitung wie im Beispiel 18 beschrieben und Chromatographie an Silicagel erhält man amorphes 17aα-(3-Jodbenzoyloxy)-D-homo-4-pregnen-3.20-dion.

UV: $\varepsilon_{222} = 35600$.

b) 172,3 mg 17aα-(3-Jodbenzoyloxy)-D-homo-4-pregnen-3.20-dion werden in 30 ml Methylenchlorid gelöst, mit 91,2 mg Jodbenzoldichlorid versetzt und mit langwelligem UV-Licht 3 Minuten bestrahlt. Anschliessend werden nochmal 91,2 mg Jodbenzoldichlorid zugefügt und weitere 3 Minuten belichtet. Nach Aufarbeitung wie im Beispiel 16 beschrieben und Schichtchromatographie erhält man nach Umkristallisation aus Aceton/Hexan das 9α-Chlor-17aα-(3-jodbenzoyloxy)-D-homo-4-pregnen-3.20-dion, das bei 210°C unter Zersetzung schmilzt.

Beispiel 21

100 mg 9α-Chlor-17aα-(3-jodbenzoyloxy)-D-homo-4-pregnen-3.20-dion werden in 12 ml Aceton und 2 ml Wasser mit 200 mg Silberperchlorat 1 Stunde zum Sieden erhitzt. Nach Aufarbeitung wie im Beispiel 17 beschrieben und Umkristallisation erhält man das 17aα-(3-Jodbenzoyloxy)-D-homo-pregna-4.9(11)-dien-3.20-dion.

Schmelzpunkt 261–263°C.

UV: $\varepsilon_{222} = 35900$.

Beispiel22

Analog Beispiel 16a–b werden 21-Acetoxy-6α-methyl-17aα-hydroxy-D-homo-4-pregnen-3.20-dion, 21-Acetoxy-6a-methyl-17aα-hydroxy-D-homo-1.4-pregnadien-3.20-dion zu 9α-Chlor-21-acetoxy-6α-methyl-17aα-(3-jodbenzoyloxy)-D-homo-4-pregnen-3.20-dion, Schmelzpunkt 198°C (Zersetzung)und

9α-Chlor-21-acetoxy-6α-methyl-17aα-(3-jodbenzoyloxy)-D-homo-pregna-1.4-dien-3.20-dion, Schmelzpunkt 209°C (Zersetzung) umgesetzt.

Beispiel23

Aus 9α-Chlor-21-acetoxy-6α-methyl-17aα-(3-jodbenzoyloxy)-D-homo-4-pregnen-3.20-dion und

9α-Chlor-21-acetoxy-6α-methyl-17aα-(3-jodbenzoyloxy)-D-homo-pregna-1.4-dien-3.20-dion werden analog Beispiel 17 21-Acetoxy-6α-methyl-17aα-(3-jodbenzoyloxy)-D-homo-pregna 4.9(11)dien-3.20-dion (Schmelzpunkt 213–215°C) und 21-Acetoxy-6α-methyl-17aα-(3-jodbenzoyloxy)-D-homo-pregna-1.4.9(11)-trien-3.20-dion (Schmelzpunkt 229-231 °C) erhalten.

Beispiel 24

a) 7,5 g 17aα-Hydroxy-D-homo-pregna-4.16-dien-3.20-dion werden in 20 ml Methylenchlorid mit 5,4 g Dimethylaminopyridin und 10,5 g 3-Jodbenzoesäureanhydrid 47 Stunden bei Raumtemperatur gerührt. Nach Aufarbeitung, Chromatographie an Silicagel und Umkristallisation aus Aceton erhält man 10,1 g 17aα-(3-Jodbenzoyloxy)-D-homo-pregna-4.16-dien-3.20-dion. Schmelzpunkt 211–212°C.

b) In einem Messzylinder werden 1950 ml Methylenchlorid 33 g Kaliumacetat und 5,05 g Phenyljoddichlorid eingetragen, Argon eingeleitet und nach Zugabe von 9,5 g 17aα-(3-Jodbenzoyloxy)-D-homo-pregna-4.16-dien-3.20-dion 15 min mit langwelligem UV-Licht bestrahlt. Zur Aufarbeitung wird mit 5%iger Natriumhydrogensulfit-Lösung, 5%iger Natriumhydrogencarbonat-Lösung und Wasser gewaschen, die Methylenchloridlösung über Natriumsulfat getrocknet und im Vakuum eingedampft. Der Rückstand wird mit Hexan verrieben und aus Aceton umkristallisiert. Man erhält so 9,65 g 9α-Chlor-17aα-(3-jodbenzoyloxy)-D-homo-pregna-4.16-dien-3.20-dion vom Schmelzpunkt 212–215°C (Zersetzung).

c) 6,68 g 9α-Chlor-17aα-(3-jodbenzoyloxy)-D-homo-pregna-4.16-dien-3.20-dion werden in 250 ml Tetrahydrofuran gelöst, 4,96 g Silberperchlorat-Monohydrat gelöst in 75 ml Wasser zugegeben und 7 Stunden unter Rückfluss erhitzt. Zur Aufarbeitung werden 10 ml Pyridin zugesetzt und im Vakuum das Tetrahydrofuran abdestilliert. Der Rückstand wird mit Methylenchlorid und Kochsalzlösung geschüttelt, die Silbersalze abfiltriert, die Methylenchlorid-Phase abgetrennt, gewaschen und eingedampft. Nach Chromatographie und Umkristallisation aus Aceton erhält man 4,85 g 17aα-(3-Jodbenzoyloxy)-D-homo-pregna-4.9(11).16-trien-3.20-dion vom Schmelzpunkt 217–218°C.

Beispiel 25

a) 420 mg 21-Acetoxy-6α-fluor-17aα-hydroxy-D-homo-4-pregnen-3.20-dion werden in 5 ml Methylenchlorid mit 978 mg Dimethylaminopyridin und 1,91 g 3-Jodbenzoesäureanhydrid werden 7 Tage bei Raumtemperatur gerührt. Nach Chromatographie an Silicagel und Umkristallisation aus Aceton/Hexan erhält man 438 mg 21-Acetoxy-6α-fluor-17aα-(3-jodbenzoyloxy)-D-homo-4-pregnen-3.20-dion vom Schmelzpunkt 228–229°C.

b) In einem Messzylinder werden 76 ml Methylenchlorid, 1,26 g Kaliumacetat und 192 mg Phenyljoddichlorid eingetragen, Argon eingeleitet, 410 mg 21-Acetoxy-6α-fluor-17aα-(3-jodbenzoyloxy)-D-homo-4-pregnen-3.20-dion zugesetzt und 5 min mit einem Ultraviolettstrahler belichtet. Nach Aufarbeitung, Verreiben des Rohproduktes mit Pentan und Umkristallisation aus Aceton erhält man 418 mg 9α-Chlor-21-acetoxy-6α-fluor-17aα-(3-jodbenzoyloxy)-D-homo-4-pregnen-3.20-dion vom Schmelzpunkt 233–234°C (Zersetzung).

c) 90,2 mg Silberperchlorat-Monohydrat werden in 5 ml Pyridin gelöst, im Vakuum eingeengt, eine Lösung von 137 mg 9α-Chlor-21-acetoxy-6α-fluor-17aα-(3-jodbenzoyloxy)-D-homo-4-pregnen-3.20-dion in 10 ml Eisessig zugegeben und 90 min auf 100°C erhitzt. Nach Aufarbeitung und Kristallisation aus Aceton/Hexan werden 114 mg 21-Acetoxy-6α-fluor-17aα-(3-jodbenzoyloxy)-D-homo-pregna-4.9(11)-dien-3.20-dion vom Schmelzpunkt 225–226°C (Zersetzung) erhalten.

Beispiel 26

a) 433 mg 21-Äthoxy-acetyloxy-17aα-hydroxy-D-homo-4-pregnen-3.20-dion werden in 5 ml Methylenchlorid mit 978 mg Dimethylaminopyridin und 1,91 g 3-Jodbenzoesäureanhydrid 7 Tage bei Raumtemperatur gerührt. Es wird aufgearbeitet, an Silicagel chromatographiert und aus Isopropyläther umkristallisiert. Man erhält so 21-Äthoxyacetyloxy-17aα-(3-jodbenzoyloxy)-D-homo-4-pregnen-3.20-dion vom Schmelzpunkt 169–171°C.

b) 400 mg 21-Äthoxy-acetyloxy-17aα-(3-jodbenzoyloxy)-D-homo-4-pregnen-3.20-dion werden analog Beispiel 24 photochloriert und aufgearbeitet. Man erhält so 380 mg 9α-Chlor-21-äthoxyacetyloxy-17aα-(3-jodbenzoyloxy)-D-homo-4-pregnen-3.20-dion vom Schmelzpunkt 183–185°C (Zersetzung).

**Patentansprüche**

1. Verfahren zur Herstellung von 17α-(3-Jodbenzoyloxy)-9α-chlor-4-pregnen-3.20-dionen und deren D-homo-analoga der allgemeinen Formel I

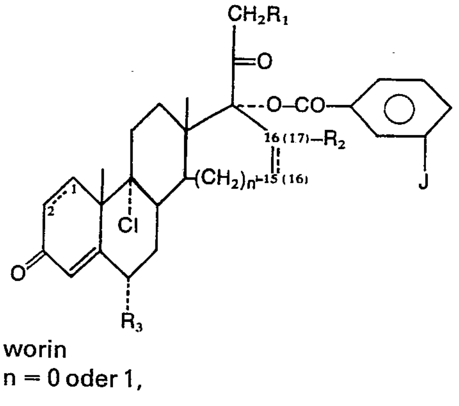

(I)

worin

n = 0 oder 1,

$C_1 \overset{\dots}{=} C_2$ eine CC-Einfach- oder CC-Doppelbindung,
$R_1$ Wasserstoff oder niederes Acyloxy,
$R_2$ Wasserstoff oder Methyl und
$R_3$ Wasserstoff, Methyl oder Fluor und falls n = 0 ist, $C_{15} \overset{\dots}{=} C_{16}$ eine CC-Einfach- oder CC-Doppelbindung und falls n = 1 ist, $C_{16} \overset{\dots}{=} C_{17}$ eine CC-Einfach- oder CC-Doppelbindung bedeuten,
dadurch gekennzeichnet, dass man 17α-Hydroxy-$\Delta^4$-3.20-ketopregnene und deren D-Homoanaloga der allgemeinen Formel II

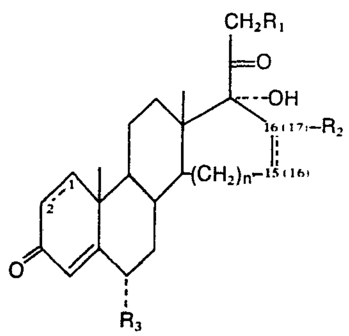
(II)

worin
$R_1$ Wasserstoff oder niederes Acyloxy und n, $C_{15} \overset{\dots}{=} C_{16}$ bzw. $C_{16} \overset{\dots}{=} C_{17}$ und $C_1 \overset{\dots}{=} C_2$, $R_2$ und $R_3$ die gleiche Bedeutung wie in Formel I haben, mit m-Jodbenzoesäure verestert und den so erhaltenen 17α-m-Jodbenzoylester in Gegenwart von Jodbenzoldichlorid mit langwelligem UV-Licht bestrahlt, gegebenenfalls in Gegenwart eines basischen Puffers.

2. 17α-(3-Jodbenzoyloxy)-9α-chlor-4-pregnen-3.20-dione und deren D-Homo-analoga der allgemeinen Formel I

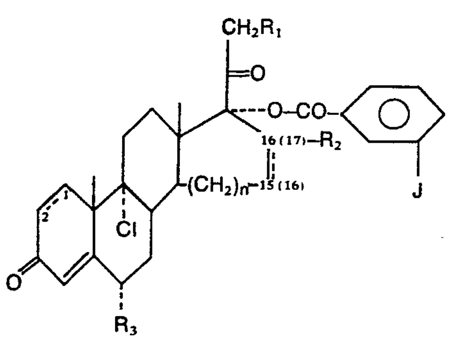
(I),

worin n = 0 oder 1,
$C_1 \overset{\dots}{=} C_2$ eine CC-Einfach- oder CC-Doppelbindung,
$R_1$ Wasserstoff oder niederes Acyloxy,
$R_2$ Wasserstoff oder Methyl und
$R_3$ Wasserstoff, Methyl oder Fluor und falls n = 0 ist, $C_{15} \overset{\dots}{=} C_{16}$ eine CC-Einfach- oder CC-Doppelbindung und falls n = 1 ist, $C_{16} \overset{\dots}{=} C_{17}$ eine CC-Einfach- oder CC-Doppelbindung bedeuten.

3. 21-Acetoxy-9α-chlor-17α-(3-jodbenzoyloxy)-4-pregnen-3.20-dion.

4. 9α-Chlor-17α-jodbenzoyloxy-4-pregnen-3.20-dion

5. 9α-Chlor-21-acetoxy-17α-(3-jodbenzoyloxy)-1.4-pregnadien-3.20-dion

6. 9α-Chlor-21-acetoxy-17α-(3-jodbenzoyloxy)-16β-methyl-1.4-pregnadien-3.20-dion

7. 9α-Chlor-21-acetoxy-17α-(3-jodbenzoyloxy)-6α-methyl-4-pregnen-3.20-dion

8. 6α-Fluor-9αChlor-21-acetoxy-17α-(3-jodbenzoyloxy)-4-pregnen-3.20-dion

9. 21-Acetoxy-9α-chlor-17aα-(3-jodbenzoyloxy)-D-homo-4-pregnen-3.20-dion

10. 9α-Chlor-17aα(3-jodbenzoyloxy)-D-homo-4-pregnen-3.20-dion

11. 9α-Chlor-21-acetoxy-17aα-(3-jodbenzoyloxy)-D-homo-1.4-pregnadien-3.20-dion

12. 9α-Chlor-21-acetoxy-17aα-(3-jodbenzoyloxy)-6α-methyl-D-homo-1.4-pregnadien-3.20-dion

13. 9α-Chlor-21-acetoxy-17aα-(3-jodbenzoyloxy)-6α-methyl-D-homo-4-pregnen-3.20-dion

14. 6α-Fluor-9α-chlor-21-acetoxy-17aα-(3-jodbenzoyloxy)-D-homo-4-pregnen-3.20-dion

15. 9α-Chlor-17aα-(3-jodbenzoyloxy)-D-homo-pregna-4.16-dien-3.20-dion

16 9α-Chlor-21-äthoxy-acetyloxy-17aα-(3-jodbenzoyloxy)-D-homo-4-pregnen-3.20-dion

## Revendications

1. Procédé de préparation d'(iodo-3 benzoyloxy)-17α chloro-9α prégnène-4 diones-3,20 et de leurs analogues D-homo, qui répondent à la formule générale I:

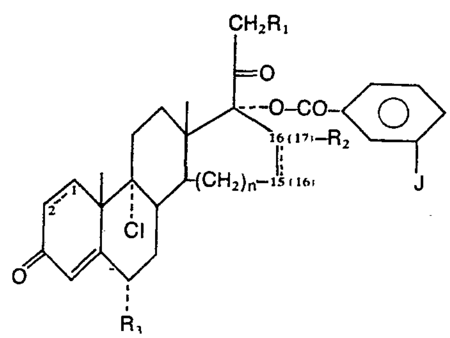
(I),

dans laquelle:
n = est égal à 0 ou à 1,
$C_1 \overset{\dots}{=} C_2$ représente une liaison carbone-carbone simple ou double,
$R_1$ représente l'hydrogène ou un radical acyloxy inférieur,
$R_2$ représente l'hydrogène ou un radical méthyle,
$R_3$ représente l'hydrogène, un radical méthyle ou le fluor,
et $C_{15} \overset{\dots}{=} C_{16}$ lorsque n est égal à 0, ou $C_{16} \overset{\dots}{=} C_{17}$ lorsque n est égal à 1, représente une liaison carbone-carbone simple ou double,
procédé caractérisé en ce qu'on estérifie au moyen de l'acide m-iodo-benzoïque des hydroxy-17α dioxo-3,20 prégnènes-4, ou leurs analogues D-homo, qui répondent à la formule générale II:

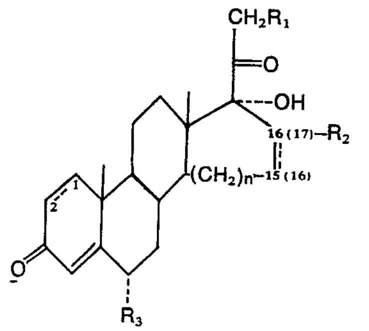

(II)

dans laquelle $R_1$ représente l'hydrogène ou un radical acyloxy inférieur et n, $C_{15}{=\!=\!=}C_{16}$ ou $C_{16}{=\!=\!=}C_{17}$ et $C_1{=\!=\!=}C_2$, $R_2$ et $R_3$ ont les mêmes significations que dans la formule I, et on irradie les esters m-iodo-benzoyliques en 17α ainsi obtenus, en présence d'iododichlorure de phényle, avec un rayonnement ultraviolet de grande longueur d'onde, éventuellement en présence d'un tampon basique.

2. (Iodo-3 benzoyloxy)-17α chloro-9α prégnène-4 diones-3,20, et leurs analogues D-homo, qui répondent à la formule I

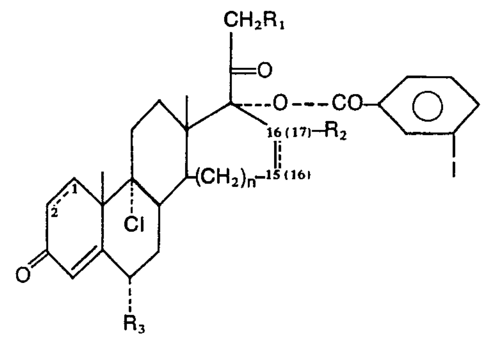

(I)

dans laquelle:
n = est égal à 0 ou à 1,
$C_1{=\!=\!=}C_2$ représente une liaison carbone-carbone simple ou double,
$R_1$ représente l'hydrogène ou un radical acyloxy inférieur,
$R_2$ représente l'hydrogène ou un radical méthyle,
$R_3$ représente l'hydrogène, un radical méthyle ou le fluor,
et $C_{15}{=\!=\!=}C_{16}$ lorsque n est égal à 0, ou $C_{16}{=\!=\!=}C_{17}$ lorsque n est égal à 1, représente une liaison carbone-carbone simple ou double.

3. Acétoxy-21 chloro-9α (iodo-3 benzoyloxy)-17α prégnène-4 dione-3,20.

4. Chloro-9α iodobenzoyloxy-17α prégnène-4 dione-3,20.

5. Chloro-9α acétoxy-21 (iodo-3 benzoyloxy)-17α prégnadiène-1,4 dione-3,20.

6. Chloro-9α acétoxy-21 (iodo-3 benzoyloxy)-17α méthyl-16β prégnadiène-1,4 dione-3,20.

7. Chloro-9α acétoxy-21 (iodo-3 benzoyloxy)-17α méthyl-6α prégnène-4 dione-3,20.

8. Fluoro-6α chloro-9α acétoxy-21 (iodo-3 benzoyloxy)-17α prégnène-4 dione-3,20.

9. Acétoxy-21 chloro-9α (iodo-3 benzoyloxy)-17aα D-homo-prégnène-4 dione-3,20.

10. Chloro-9α (iodo-3 benzoyloxy)-17aα D-homo-prégnène-4 dione-3,20.

11. Chloro-9α acétoxy-21 (iodo-3 benzoyloxy)-17aα D-homo-prégnadiène-1,4 dione-3,20.

12. Chloro-9α acétoxy-21 (iodo-3 benzoyloxy)-17aα méthyl-6α D-homo-prégnadiène-1,4 dione-3,20.

13. Chloro-9α acétoxy-21 (iodo-3 benzoyloxy)-17aα méthyl-6α D-homo-prégnène-4 dione-3,20.

14. Fluoro-6α chloro-9α acétoxy-21 (iodo-3 benzoyloxy)-17aα D-homo-prégnène-4 dione-3,20.

15. Chloro-9α (iodo-3 benzoyloxy)-17aα D-homo-prégnadiène-4,16 dione-3,20.

16. Chloro-9α éthoxyacétyloxy-21 (iodo-3 benzoyloxy)-17aα D-homo-prégnène-4 dione-3,20.

## Claims

1. A process for the preparation of a 17α-(3-iodobenzoyloxy)-9α-chloro-4-pregnene-3,20-dione or a D-homo homolog thereof of the formula I

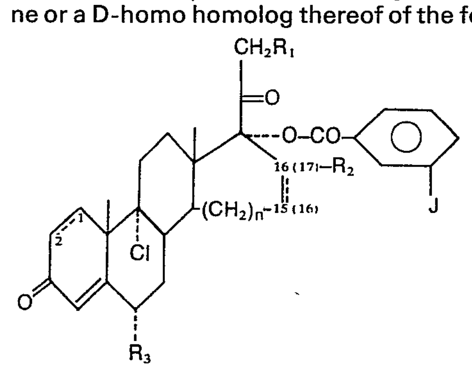

(I),

wherein
n is 0 or 1;
$C_1{=\!=\!=}C_2$ represents a C-C single or C=C double bond;
$R_1$ is hydrogen or lower alkanoyloxy;
$R_2$ is hydrogen or methyl;
$R_3$ is hydrogen, methyl or fluorine; and
when n is 0, $C_{15}{=\!=\!=}C_{16}$ represents a C-C or C=C double bond, and when n is 1, $C_{16}{=\!=\!=}C_{17}$ represents a C-C single or C=C double bond,

which comprises esterifying a 17α-hydroxy-4-pregnene-3,20-dione or a D-homo homolog thereof of the formula II

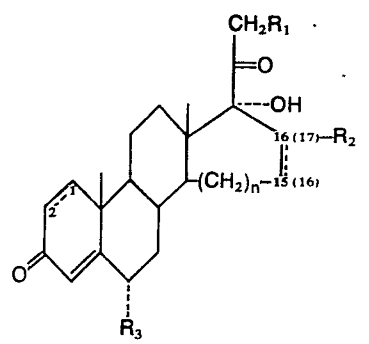

(II),

wherein $R_1$ is hydrogen or lower alkanoyloxy, and n, $C_{15}{=\!=\!=}C_{16}$ or $C_{16}{=\!=\!=}C_{17}$ and $C_1{=\!=\!=}C_2$, $R_2$ and $R_3$ have the meaning as given in formula I, with m-iodobenzoic acid, irridiating the thus produces 17α-m-iodobenzoic acid ester with long wavelength ultraviolet radiation in the presence of

phenyliodine dichloride, optionally in the presence of a basic buffer.

2. A 17α-(3-iodobenzoyloxy)-9α-chloro-4-pregnene-3,20-dione or a D-homo homolog thereof of the formula I

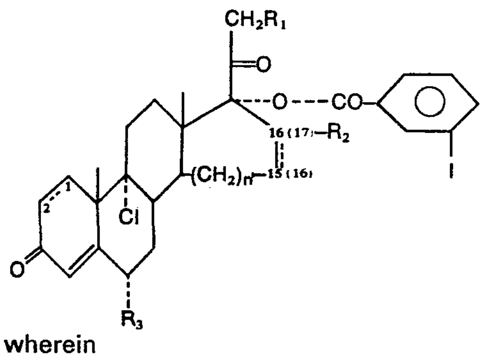

(I)

wherein

n is 0 or 1;

$C_1 === C_2$ represents a C-C single or C=C double bond;

$R_1$ is hydrogen or lower alkanoyloxy;

$R_2$ is hydrogen or methyl;

$R_3$ is hydrogen, methyl or fluorine; and

when n is 0, $C_{15} === C_{16}$ represents a C-C or C=C double bond, and when n is 1, $C_{16} === C_{17}$ represents a C-C single or C=C double bond.

3. 21-Acetoxy-9α-chloro-17α-(3-iodobenzoyloxy)-1-4-pregnene-3,20-dione;

4. 9α-Chloro-17α-(3-iodobenzoyloxy)-4-pregnene-3,20-dione;

5. 9α-Chloro-17α-(3-iodobenzoyloxy)-4-pregnene-3,20-dione;

6. 9α-Chloro-21-acetoxy-17α-(3-iodobenzoyloxy)-16β-methyl-1,4-pregnadiene-3,20-dione, a compound of claim 1;

7. 9α-Chloro-21-acetoxy-17α-(3-iodobenzoyloxy)-6α-methyl-4-pregnene-3,20-dione;

8. 6α-Fluoro-9α-chloro-21-acetoxy-17α-(3-iodobenzoyloxy)-4-pregnene-3,20-dione;

9. 21-Acetoxy-9α-chloro-17aα-(3-iodobenzoyloxy)-D-homo-4-pregnene-3,20-dione;

10. 9α-Chloro-17αa-(3-iodobenzoyloxy)-D-homo-4-pregnene-3,20-dione;

11. 9α-Chloro-21-acetoxy-17aα-(3-iodobenzoyloxy)-D-homo-1,4-pregnadiene-3,20-dione;

12. 9α-Chloro-21-acetoxy-17aα-(3-iodobenzoyloxy)-6α-methyl-D-homo-1,4-pregnadiene-3,20-dione;

13. 9α-Chloro-21-acetoxy-17aα-(3-iodobenzoyloxy)-6α-methyl-D-homo-4-pregnene-3,20-dione;

14. 6α-Fluoro-9α-chloro-21-acetoxy-17aα-(3-iodobenzoyloxy)-D-homo-4-pregnene-3,20-dione;

15. 9α-Chloro-17aα-(3-iodobenzoyloxy)-D-homo-pregna-4,16-diene-3,20-dione;

16. 9α-Chloro-21-ethoxyacetyloxy-17aα-(3-iodobenzoyloxy)-D-homo-4-pregnene-3,20-dione.